# EUROPÄISCHE PATENTSCHRIFT

(11) **EP 1 327 886 B1**
(45) Veröffentlichungstag und Bekanntmachung des Hinweises auf die Patenterteilung: **18.05.2005**
(21) Anmeldenummer: 02000676.3
(22) Anmeldetag: 11.01.2002
(51) Int. Cl.: G01N 33/558, G01N 33/543

(54) **Verfahren zum Nachweis von Analyten in Proben mittels Analysenelementen**
Method for detecting analytes with analytical elements
Procédé pour la détection d'analytes par élements pour l'analyse

(43) Veröffentlichungstag der Anmeldung: 16.07.2003
(73) Patentinhaber: 8 sens biognostic AG, 13125 Berlin (DE)
(72) Erfinder: Renneberg, Reinhard Prof. Dr., Clear Water Bay, Kowloon, SAR Hong Kong (CN); Lehmann, Karin, 13125 Berlin (DE); Dr. Lehmann, Matthias, 13125 Berlin (DE)
(74) Vertreter: Baumbach, Friedrich, Dr.

(56) Entgegenhaltungen:
- EP-A- 0 427 534
- EP-A- 0 696 735
- DE-A- 19 940 394
- US-A- 5 786 220
- US-A- 5 962 339
- US-B1- 6 197 881
- US-B1- 6 329 145
- SELLERGREN B & ANDERSSON LI: "Application of imprinted synthetic polymers in binding assay development." METHODS, Bd. 22, 2000, Seiten 92-106, XP002201794

## Beschreibung

Die Erfindung betrifft ein Verfahren zum Nachweis von zahlreichen Analyten, wie z.B. von Antigenen, Antikörpern, Rezeptorliganden, Rezeptoren, DNA und RNA in biologischen oder anderen flüssigen Proben, mittels Analysenelementen, insbesondere als Teststreifen, wobei das Verfahren auf Affinitätsreaktionen beruht.
Bevorzugt betrifft sie den Nachweis von Antigenen. Dabei handelt es sich im Sinne der Erfindung beispielsweise um hochmolekulare Antigene, wie z.B. Proteine, oder niedermolekulare Haptene, wie z.B. Pestizide, andere Schadstoffe oder Hormone.
Desweiteren betrifft die Erfindung entsprechende Analysenelemente an sich. Die Ergebnisse können direkt aus dem Assay ermittelt werden. Anwendungsgebiete der Erfindung sind vor allem die medizinische Diagnostik, die pharmazeutische Industrie und der Umweltschutz.

Analytische Elemente, wie z.B. Teststreifen (Laterale Immuno-Dipsticks), zum Nachweis von zahlreichen Analyten in biologischen Flüssigkeiten oder zum Nachweis von Schadstoffen in Umweltproben sind an sich bekannt. Sie werden prinzipiell entweder nach dem Sandwich- oder nach dem Konkurrenzprinzip bestimmt.

Die Verwendung von Affinitätsassays, zum Beispiel Immunoassays, Rezeptor- und DNA-Assays, ist von immer größerer Bedeutung für klinische und zahlreiche andere Anwendungen wie beim Umweltschutz.

Sehr gebräuchlich ist der "Immuno-Lateral-Flow-Test". Der bei dieser Untersuchung angewendete Assay setzt sich aus vier Teilen auf einem Teststreifen zusammen: einem Probenaufgabefeld, einem Konjugatfeld, einem Analysenfeld (üblicherweise eine Nitrocellulosemembran) und einem Absorptionsfeld. Die Probenflüssigkeit fließt durch das Probenfeld mittels Kapillarkräfte in das Konjugatfeld. In und auf dem Konjugatfeld sind markierte Antikörper (z.B. mit kolloidalem Gold markiert, was eine Rotfärbung ergibt, oder mit Enzymen) gespeichert. Die Probenflüssigkeit fließt durch dieses Konjugatfeld und löst somit die markierten Antikörper ab. Die Probenlösung fließt dann, die markierten Antikörper und den Analyten mitführend, von dem Konjugatfeld in das Analysenfeld, auf dem Fängerantikörper adsorbiert sind. Schließlich fließt sie durch die Membran des Analysenfeldes zum Absorptionsfeld. Zusammen bilden sie den Teststreifen.
Gewöhnlich sind bei einem Assay für hochmolekulare Antigene zwei immun-reaktive Linien auf dem Analysenfeld fixiert: eine für die Feststellung des interessierenden Analyten (z.B. Antikörper gegen einen Herzinfarktsmarker wie FABP) und eine dahinter gelegene Linie als positive Kontrolle für den Test (z.B. Antimaus-Antikörper). Der Teststreifen kann sich in einem zusätzlichen Plastikhalter befinden oder direkt in die Flüssigkeit getaucht werden. In einem Teststreifen für mehr als einen Parameter vergrößert sich dieser doppelte Bindebereich um die Zahl der Analyten, die mehr gemessen werden, z.B. gibt es für 3 Analyten drei Bindebereiche, die für den Analyten spezifisch sind, und einen zur Kontrolle, dass die verwendeten Antikörper intakt sind. Ist kein Analyt in der Probe enthalten, fließen die markierten Antikörper ungehindert zum Absorptionsfeld und binden lediglich am Kontrollantikörper. Über die rote Farbe des kolloidalen Goldmarkers zeigen sie, daß der Assay prinzipiell funktioniert. Ist dagegen Analyt (z. B. FABP) in der Probe, bindet er zürst im Konjugatfeld an die goldmarkierten Antikörper und fließt mit ihnen in die Analysenfeld. Dort bindet der Komplex aus Analyt (FABP) und markierten Antikörpern an die fixierten Fängerantikörper gegen den Analyten (FABP). Der nichtgebundene Rest der markierten Antikörper (ohne Analyt) bindet an die dahinter gelegenen Kontrollantikörper. Die zwei roten Linien zeigen somit das Analytsignal und das Kontrollsignal.

Ähnliche Assays sind für Rezeptoren und DNA möglich, wenngleich auch noch nicht weitpraktiziert wie bei Immunoassays.

Eine Vielzahl von Immuntests ist heute verfügbar (Übersicht in P.Tijssen: Practice and *Theory of Enzyme Immunoassays*, Elsevier Amsterdam 1985, S.9-20; D.S. Hage, *Immunoassays.* Analytical Chemistry 71 (12) 1999, S. 294R-304R). Sie alle nutzen die einzigartigen Eigenschaften der Antikörper, die spezifische Erkennung und Bindung von Molekülen, zur Analyse. Ähnliches gilt für andere Affinitätsassays wie DNA-Assays. Hier werden meist an eine Oberfläche fixierte DNA-Bruchstücke durch Oligonucleotid-Sonden (probes) erkannt, die sich binden und markiert sind.

Ein konkretes Beispiel soll den Stand der Technik erläutern: Ein wichtiges Anwendungsgebiet für diese Assays ist die Diagnose von Herzinfarkten. Während das klassische EKG nur in etwa 40-50 % der Fälle bei Ankunft im Krankenhaus eine klare diagnostische Aussage trifft, kann ein "früher" biochemischer Herzinfarkt-Marker wie Fatty Acid-Binding Protein (FABP) die Frühdiagnose wesentlich verbessern (Renneberg, R., Cheng, S., Kaptein, W.A., McNeil, C.J., Rishpon, J., Gründig, B., Sanderson, J., Chu, A., Glatz, J.F.C. *Novel immunosensors for rapid diagnosis of acute myocardial infarction: A case report,* Advances in Biosensors ed. A.P.F. Turner, R. Renneberg, Biosensors: a Chinese perspective, Vol 4 (1999).)

Die bisher verfügbaren Tests für "späte" Herzinfarkt-Marker sind jedoch entweder Labortests, die somit gutausgebildetes Personal, teuere Apparate und eine lange Verweilzeit erfordern, oder es sind Schnelltests. Diese messen verschiedene Markermoleküle mit einem kalibrierten Lesegerät quantitativ (z.B. den "späten" Marker Troponin T - EP 00394819 A2) oder qualitativ (z.B. Jackowski, George /US-A 5,604,105).
Die bisherigen visuell ausgewerteten Sandwich-Tests für hochmolekulare Ag funktionieren wie folgt: Wenn der Analyt (hier ein Ag) in entsprechender Probenflüssigkeit (z.B. Plasma) im bis dahin trockenen Auftragefeld appliziert wird, bindet er an lose aufgetragene Detektorantikörper (DAk, die ihrerseits einen Markerstoff (M) gebunden haben. Der Antigen-Detektorantikörper-Marker-Komplex (Ag-DAk-M) bewegt er sich mit der Trägerflüssigkeit durch kapillare Kräfte in Richtung der Nachweisfeldes und der Ag-DAk-M bindet sich an dort fixierten Fängerantikörpern (FAk) über das Ag. Es entsteht ein Sandwich-Komplex, der zumeist als farbige Linie sichtbar ist und anzeigt, dass der Analyt in der Probe enthalten war.
Die farbige Linie ist dabei der Analytkonzentration proportional. Eine Analytkonzentration einer gesunden Person, die normale Cardiomarker-Mengen enthält, zB. 10 ng FABP pro mL, ruft also beispielsweise eine Färbung hervor, die z.B. etwa 20mal schwächer intensiv ist als die Färbung, die durch Probe eines Herzinfaktpatienten mit 200 ng/mL erzeugt wird. Der Testende kann aber die kaum sichtbare schwache farbige Linie beim Gesunden durchaus subjektiv als eine positive Probe interpretieren oder wird verunsichert.
10 ng FABP/mL im Blut sind also ein Normalwert. Bei Angina pectoris steigt der Wert auf etwa 50ng/mL, bei Herzinfarkt dagegen weit darüber. Da die Farbentwicklung der Tests linear mit der Analytmenge steigt, zeigt ein Test für FABP auch schon bei Normalwerten wie 10 ng/mL eine sehr schwache, aber doch sichtbare Linie an. Das kann, besonders da es um Leben und Tod geht, durchaus als POSITIVES SIGNAL fehlgedeutet werden. Das führt zur Verunsicherung des Testenden. Dieser nimmt an, dass diese schwache Linie ein positives Signal (also z.B. das Vorliegen eines Herzinfarktes) bedeutet. Auch starkes Muskeltraining kann den Wert der Cardiomarker im Plasma zusätzlich, wenn auch geringfügig, erhöhen. Dadurch liefert der Test einen falschpositiven Wert
Wünschenswert wäre also, den Analyten erst nach Erreichen des Schwellenwertes anzuzeigen, dass der Test für Gesunde, also bis 10 ng FABP/mL, KEINERLEI Färbung zeigt. Das konnte bisher nur dadurch erreicht werden, dass der gesamte Test unempfindlicher gestaltet wurde

Die bekannten Tests nach dem Konkurrenzprinzip funktionieren dagegen anders: Aufgrund der geringen Größe des Ag (meist unter MW 1,000, auch Hapten genannt) kann nur ein Akbinden, ein Sandwich kann also nicht konstruiert werden. Es gibt also keine markierten Detektor-Ak. In diesem Fall wird eine definierte Menge an Ag mit Markern gelabelt (Ag-M) und bewegt sich gleichzeitig mit dem nachzuweisenden Ag und der Probeflüssigkeit kapillar zu den Fänger-Ak im Nachweisfeld. Dort konkurrieren eine definierte Menge markierter und unmarkierte (nachzuweisende) Ag um die Bindung an den dort fest gebundenen FAk. Wenn kein Ag in der Probe war, binden die FAk nur markierte (gefärbte) Ag und zeigen eine gefärbte Linie, war dagegen eine grosse Ag-Menge in der Probe, bindet sich fast ausschliesslich (ungefärbtes) Ag aus der Probe, es erscheint keine Farblinie. Diese Gegenläufigkeit (Je mehr Signal, desto weniger Analyt in der Probe) führt oft zu Missverständnissen beim Testenden, vor allem bei Laien. Wünschenswert ist, dass das Vorliegen eines Analyten in der Probe nicht nur zu einem proportionalen Farbsignal führt, sondern Normalwerte an Antigen auch nicht angezeigt werden.

Aus EP 0 833 159 A2 (US-A 5,798,273) sind Teststreifen (lateral-flow assays) zur Detektion kleiner Antigene (am Beispiel von PCB) nach dem Konkurrenzprinzip bekannt, die aus drei Zonen bestehen: 1. eine Auftragezone zum Auftragen der Probe und von anti-Analyt-Antikörpern mit Farbpartikeln markiert und unabhängigen Tracern, 2. ein capture-Bereich (Zone 2) mit Analyt und Analyt-Analogen, die an Partikeln immobilisiert sind, und 3. ein red-out Bereich mit einer und mehr Linien von Anti-Analyt-Antikörpern. Jede Zone hat somit unterschiedliche Beschichtungen. Wenn eine Probe keinen Analyten enthält, binden die markierten Antikörper aus der Auftragezone in Zone2, wenn dagegen Analyt vorhanden ist, bindet er sofort an die Antikörper und nicht mehr in Zone 3 und wird in Zone 3 sichtbar. So können mit Erhöhung der Analytkonzentration auch eine Erhöhung des Signals erreicht werden.

Der Erfindung lag die Aufgabe zugrunde, einen schnellen und zuverlässigen Assay zur Untersuchung von vorzugsweise biologischen Materialien zu entwickeln, der einfach zu handhaben ist und falschpositive Ergebnisse verhindert, die entstehen können, weil in der Probe auch bei einem Normalwert bereits Analyt in geringer Menge vorhanden ist und als schwaches Signal angezeigt wird. Es sollen Verfahren und Analysenelemente bereitgestellt werden, die eine unverfälschte Nachweisreaktion von Analyten ermöglichen, wobei lediglich Werte angezeigt werden sollen, die über einem vorhandenen Normalwert oder anderem festgesetzten Wert liegen.

Folgende Dokumente gehören ebenfalls noch zum Stand der Technik:
US 5 962 339 beschreibt ein immunochemisches Verfahren zum Nachweis von Antigenen und Antikörpern. Dabei wird eine Schwellenregion bestehend aus einem zweiten bindenden Agenz benutzt, welches einen bestimmten Teil nichtkomplexiertes erstes bindendes Agenz zurückhält (Auszug aus Anspruch 1, Sp. 6, Z. 34). Um Antikörper als Analyten zu detektieren, werden in der Probenzone farbstoffgelabelte Antigene (erstes bindendes Agenz) gegen den Antikörper platziert. Wenn die nachzuweisenden Antikörper in einer geringen Konzentration in der Probe vorkommen, binden nicht alle farbstoffgelabelten Antigene mit dem Antikörper. Der Überschuss bindet sich an anti-Antikörper (zweites bindendes Agenz) der Schwelle und es erscheint kein Signal in der Nachweiszone. Überschreitet die Konzentration der Antikörper jedoch einen bestimmten Wert, blockieren die Antikörper-Analyte alle farbstoffgelabelten Antigene in der Probenzone. Dadurch binden an der Schwelle keine gelabelten Antigene, weil das zweite bindende Agenz gegen das erste bindende Agenz (Antigene) gerichtet ist. Dafür binden aber die entstandenen Antikörper-Antigene (farbstoffgelabelt) in der Nachweiszone und geben dort ein farbiges Signal. Somit wird das Überschreiten einer Analytkonzentration angezeigt.
Bei EP 0 696 735 wird ein immunochromatographischer Test mit kontrollierter Sensitivität erläutert, der sich auf den Nachweis hochmolekularer Analyte (am Beispiel von Hemoglobin) und das Sandwich-Prinzip beschränkt. Dabei werden ungelabelte Antikörper gegen den Analyten bereits in der Probenauftragszone durch Auftrocknen lose deponiert. Überschreitet die Analyt(Antigen)konzentration einen bestimmten Wert, dann bewegen sich nicht-gebundene Antigene über die Konjugatzone, wo sie mit einem gelabelten Antikörper Komplexe bilden, zur Nachweiszone und erzeugen dort ein farbiges Signal.
US 5 786 220 beschreibt einen immunochromatographischen Test, der Werte von Progesteron und hCG unterhalb bzw. oberhalb eines festgelegten Normalwertes (Schwellenwertes) als farbige Streifen anzeigt. Die Analyten, oder ihre Derivate, auf dem Streifen werden dabei farbig markiert und konkurrieren mit den nicht-markierten nachzuweisenden Analyten aus der Probe nach dem Konkurrenz- oder dem Verdrängungsprinzip um die Bindungsstellen. Nur wenn die Analytkonzentration in der Probe einen Schwellenwert überschreitet, werden die farbig gelabelten Analytmoleküle in der Nachweiszone gebunden.
EP 0 427 534 beschreibt einen Teststreifen (am Beispiel eines enzymatischen Cholesteroltests), der vorzugsweise enzym-generierte Signale anzeigt, deren Distanz vom Auftragungsort der Probe proportional zur Menge des Analyten ist. Dabei werden keine Schwellenaffinitätsfänger verwendet.
US 6 329 145 beschreibt die Bestimmung von Substanzen, die an Nukleinsäuremoleküle binden. Durch die Bindung wird eine farbstoff-gelabelte Nukleinsäure verdrängt und anschließend detektiert. Das Aptamere als Substanzen für solche Bindungen nutzbar sind, ist nicht neu, sondern gehört zum Stand der Technik.
Der Review des Dokuments Methods 22 (2000): 92 - 106 beschreibt, wie Imprinted Polymere synthetisiert und zur Analytik verwendet werden können. Dabei werden Imprinted Polymere als mögliche Substanzen zur Erkennung genannt.
DE 199 40 394 beschreibt Fluorophore, die für die Detektion von verschiedenen Substanzen als Label nutzbar sind.
US 6 197 881 befasst sich mit elektrochemisch aktiven Copolymeren und deren Herstellung. Dabei werden die Copolymere über Streptavidin-Biotin an Oberflächen gebunden.

Die Erfindung wird gemäß den Ansprüchen realisiert. Das erfindungsgemäße Verfahren ist universell verwendbar und verläuft auf der Basis von Affinitätsreaktionen in analytischen biologischen oder anderen flüssigen Proben, wie z.B. Blut- und Serumproben oder Umweltproben, mittels Analysenelementen, insbesondere als Teststreifen. Es ist z.B. zum Nachweis zahlreicher Analyten, wie z.B. von Antigenen, Antikörpern, Rezeptorliganden, Rezeptoren, DNA und RNA geeignet. Bevorzugt betrifft es den Nachweis von Antigenen. Dabei handelt es sich im Sinne der Erfindung beispielsweise um hochmolekulare Antigene, wie z.B. Proteine oder niedermolekulare Haptene, wie z.B. Pestizide, Schadstoffe oder Hormone.

Am Beispiel von Immunoassays wird die Idee der Erfindung im folgenden erläutert. Anstelle von Antigenen und Antikörpern können auch Liganden und Rezeptoren, Lectine, Protein A und G, verschieden Formen von DNA und RNA verwendet werden.

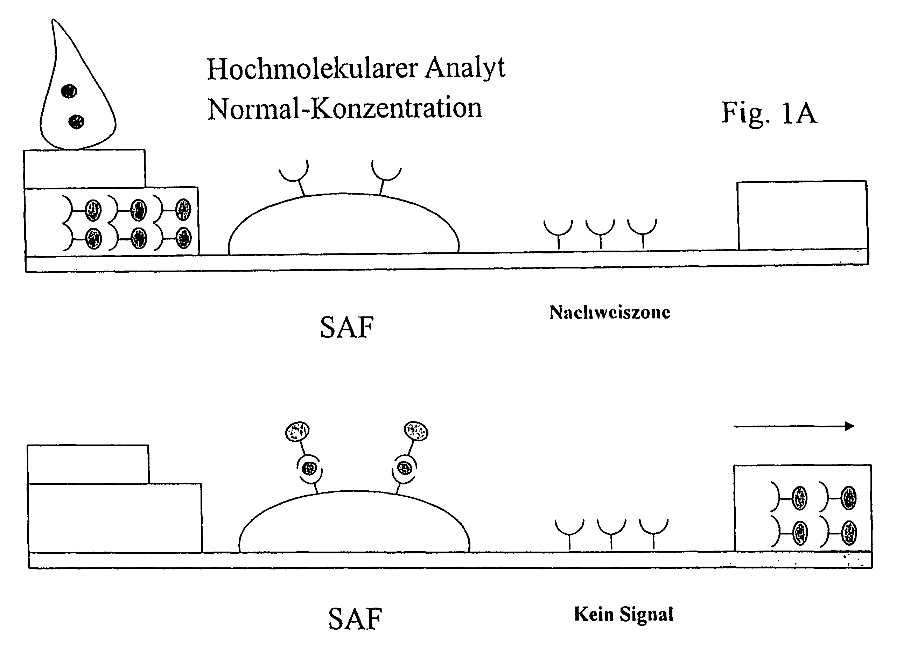

Das Verfahren ist dadurch gekennzeichnet, dass zusätzlich zu gebräuchlichen Beschichtungen und -konfigurationen mit komplementären Affinitätsmolekülen in der Nachweiszone das Analysenelement zwischen Probeaufnahmezone und Nachweiszone mindestens eine Fängersubstanz als Schwellen-Affinitäts-Fänger (SAF engl. treshold affinity catcher) sichtbar oder verdeckt - für eine vorgelagerte Affinitäts-Reaktion aufweist, in einer Konzentration, die den Normalwert des zu bestimmenden Analyten eines Gesunden, einer Umweltprobe oder einen anderen festgelegten Wert eliminiert, wobei die Fängersubstanzen auf dem Analysenelement räumlich so angeordnet sind, dass diese Reaktion der Nachweisreaktion als Schwelle vorgelagert abläuft und somit erst Werte über dem Schwellenwert in der Nachweisreaktion sichtbar werden.
Durch der Nachweisreaktion vorgelagerte vorzugsweise nichtsichtbare (verdeckte) Immunreaktionen ("Immunoschwelle") wird beim Sandwichprinzip das gegen einen Analyten gerichtete Fängermolekül (der Schwellen-Affinitäts-Fänger (SAF)) in bestimmter Konzentration räumlich und zeitlich gesehen vor der Nachweisreaktion an entsprechenden Trägermaterialien fest gebunden (vgl. Abb.1A)
Erfindungsgemäß wird die Analyt-Menge (A), die einem Normalwert oder sonstigem festgelegtem Wert (im folgenden "Analyt-Schwellenwertmenge" genannt) durch eine "Immunoschwelle" nach dem Titrationsprinzip gebunden und erreicht so nicht mehr die Fänger-Affinitätsmoleküle im Nachweisfeld.
Die Immunoschwelle besteht aus direkt oder über Trägerproteine fest gebundene Fängermoleküle definierter Konzentration, die so dosiert sind, dass sie sicher genau die Mengen an einen Analyt-Detektor-Affinitätsmolekül-Marker-Komplex (A-Dam-M-Komplex) binden, die der festgelegten Analyt-Schwellenwertmenge entsprechen. Wenn also beispielsweise 20ng FABP/mL in einer 100microliter Plasma-Probe enthalten sind, werden die absolute Menge von 2 ng FABP, mit den entsprechen Dam-M gebunden, bereits an der Immunoschwelle abgefangen und kann nicht das Nachweisfeld erreichen.

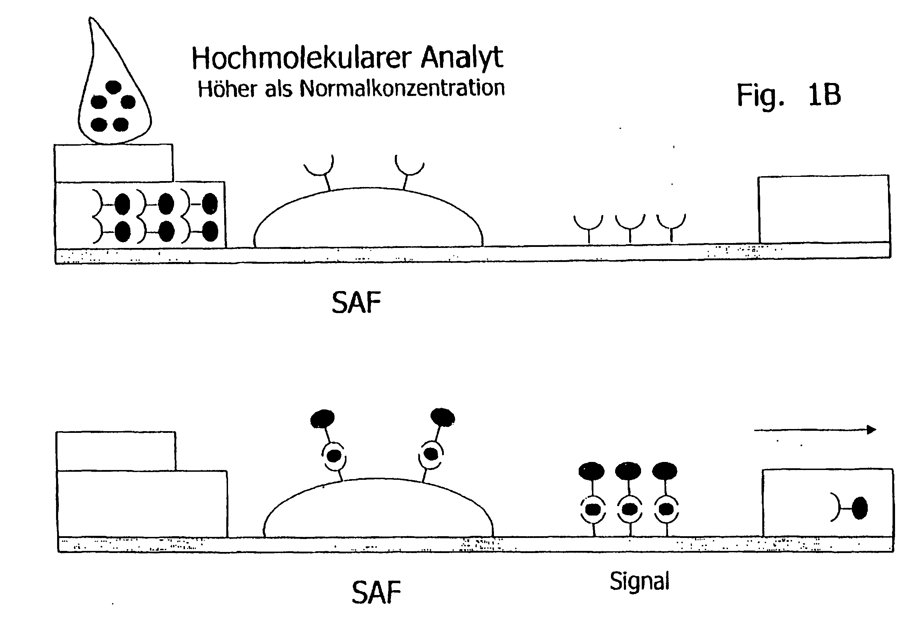

Bevorzugt ist die Immunoschwelle verdeckt und kann leicht mit einem Teststreifengehäuse abgedeckt werden, das nur das echte Testfeld als Fenster zeigt. Der Teststreifen zeigt in diesem Fall erst eine farbige Linie an, wenn höhere Konzentrationen als 20 ng/mL in der Probe enthalten sind (Abb.1B)

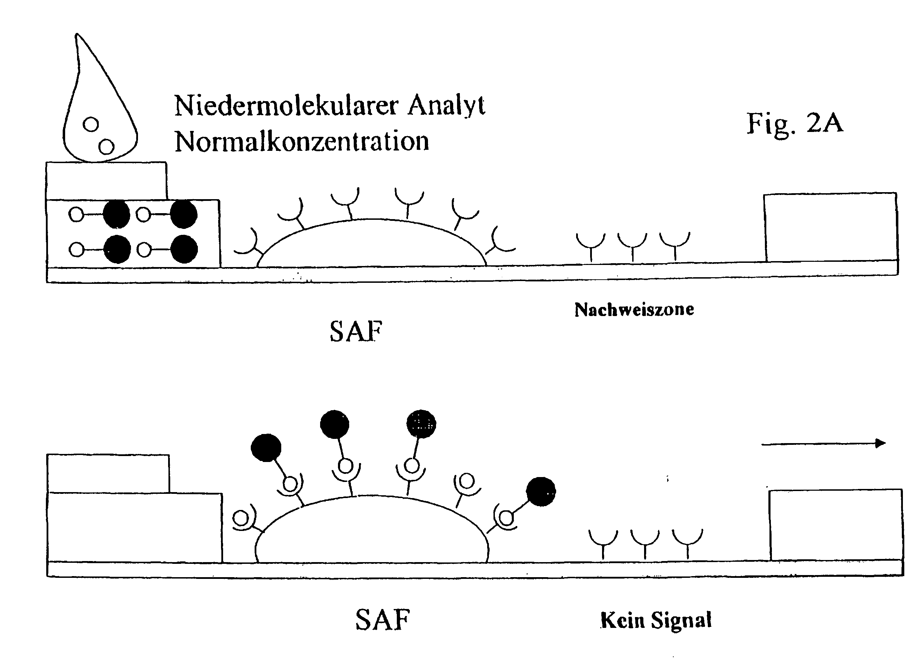

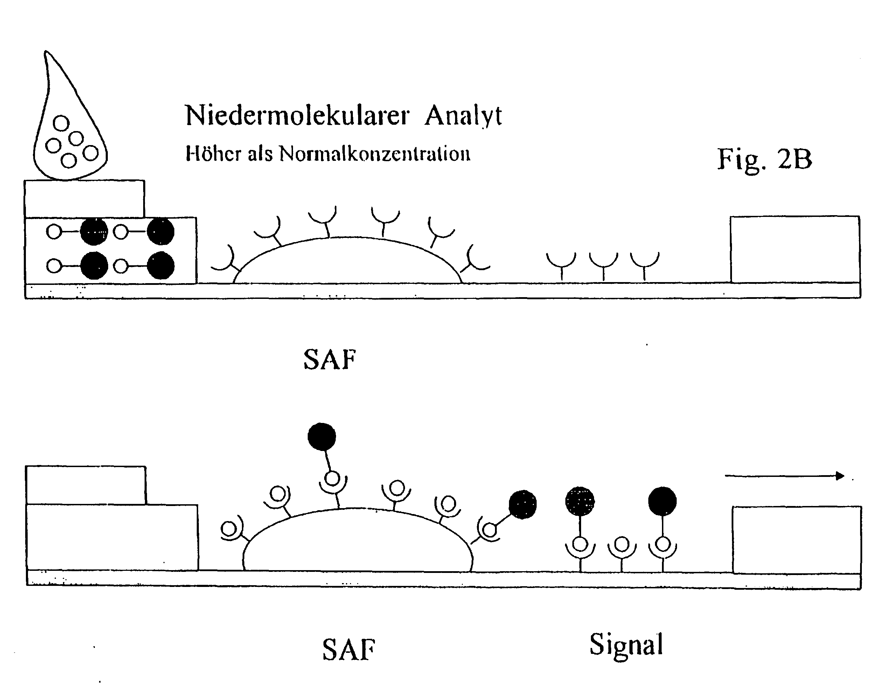

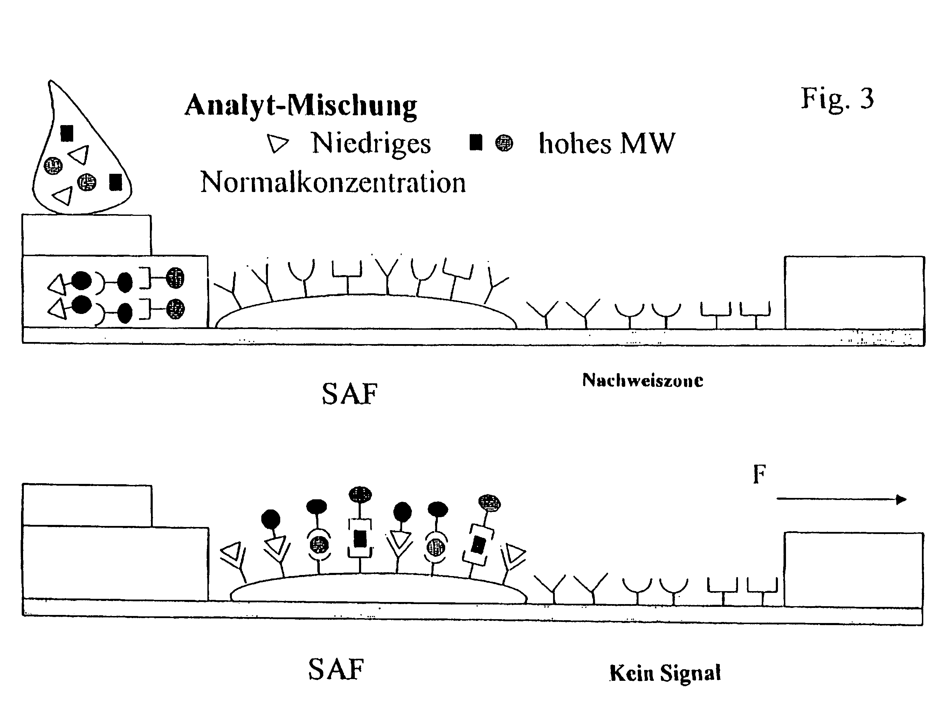

Erfindungsgemäß wird für den Nachweis niedermolekularer Analyte wie es z.B. Haptene sind, dem Nachweisfeld ebenfalls eine bevorzugt verdeckte, allerdings anders konstruierte Immunoschwelle vorgelagert. Diese vorgelagerte Immunoschwelle besteht vorzugsweise aus fest gebundenen Fänger-Ak als SAF und ist so konstruiert, dass bei Normalkonzentration eines Analyten in der Probe sämtliche A-M und A-Moleküle in der Immunoschwelle verbleiben. In das Nachweisfeld gelangt somit kein A-M und es bleibt ohne gefärbte Linie (Abb.2A). Bei hoher A-Menge in der Probe, also über der Schwellenkonzentration, gelangt ein proportionaler Teil der (gefärbten) A-M in das Nachweisfeld (Abb.2 B)

Antikörper und Antigene stehen als Beispiele für andere Affinitätsmoleküle, z. B. können verschiedene monoklonale oder polyklonale Antikörper oder deren Fragmente oder Fusionsprodukte eingesetzt werden, Avidin, Streptavidin und Biotin, Protein A und G, Lectine, Rezeptoren und Rezeptorliganden, ein- und doppelsträngige DNA oder RNA oder deren Fragmente, Aptamere oder sogenannte imprinted polymers.

Bevorzugte Materialien für die Teststreifen sind verschiedene Cellulosemembranen, aber auch Glasfiber, Nylon, andere Flüssigkeitsträger, wie Papier, Baumwolle oder teststreifenähnliche Strukturen aus Glas, Silicon, Plastik, Metall mit eingebrachten Kanälen oder poröse Materialien.

Die erfindungsgemäßen lateral-flow Assays (Teststreifen) können mit identischen oder unterschiedlichen Affinitätsmolekülen präpariert sein, die auf der Oberfläche in Linien, Punkten oder anderen geometrische Flächen verschiedener Größe aufgetragen sind, die durch fixierte Affinitätsmoleküle gebildet werden. Variationen je nach konkreter Aufgabe sind auch bezüglich der Konzentration der Affinitätsmoleküle per Linie oder einer andere geometrische Fläche möglich. Die Fängersubstanzen befinden sich vorzugsweise direkt gebunden über Spacersubstanzen oder Trägerproteine auf dem Analysenelement und können in unterschiedlichen geometrischen Strukturen vor liegen, wie z.B. in Strichform, Kreisform, als Punkte, Ellipsen oder anderen geometrischen Figuren. Um den Benutzer nicht zu irritieren, sind sie bevorzugt verdeckt (unsichtbar) auf dem Analysenelement angeordnet.

Es können SAF sowohl für einen Analyten oder aber auch für mehrere Analyten in der Schwelle enthalten sein. Das hat den Vorteil, dass auch Substanzen eliminiert werden können, die in der Nachweiszone stören würden.

In einer bevorzugten Ausführungsvariante der Erfindung handelt es sich um ein Verfahren zum Nachweis von Antigenen, welches dadurch gekennzeichnet ist, dass zusätzlich zu gebräuchlichen Immunobeschichtungen und -konfigurationen das Analysenelement als SAF mindestens einen Fängerantikörper (Schwellen-FAk) für eine vorgelagerte Antigen-Antikörper-Reaktion aufweist, in einer Konzentration, die den Normalwert des Antigens eines Gesunden, einer Umweltprobe oder einen anderen festgelegten Wert eliminiert, wobei die FAk auf dem Analysenelement räumlich so angeordnet sind, dass diese Reaktion der Nachweisreaktion als Immunoschwelle (engl. Immuno treshold) vorgelagert abläuft. Diese FAk werden im folgenden Schwellen-FAk (treshold catcher antibodies) genannt.

In einer besonderen Ausführungsvariante der Erfindung verläuft das Verfahren zum Nachweis von höhermolekularen Analyten, bevorzugt von Antigenen (Ag), wie z.B. Proteine, nach dem Sandwichprinzip im Detail wie folgt:

Das Analysenelement umfasst in der Probenaufnahmezone frei bewegliche Detektoraffinitätsmoleküle (Dam), für den Antigennachweis Detektorantikörper (DAk), welche mit einem Marker (M) zur Signalbildung chemisch fest verbunden in trockener Form vorliegen. Nach dem Auftragen der zu untersuchenden flüssigen Probe bilden sie mit dem zugefügten Analyten einen Analyt-Detektorantikörper-Marker-Komplex (A-Dam-M), welcher sich durch kapillare Kräfte in Richtung Nachweisfeld, das ebenfalls die Fängermoleküle, die gegen den Analyten gerichtet sind, für die Nachweisreaktion umfasst, bewegt,

Der Komplex passiert dabei die SAF, die gegen einen zu untersuchenden Analyten gerichtet sind und die direkt über Spacersubstanzen oder über Trägerproteine an das Analysenelement fest gebunden vorliegen. Die SAF weisen eine definierte Konzentration auf, so dass sie sicher genau die Menge an A-Dam-M binden, welcher dem Normalwert des Analyten eines Gesunden, einer Umweltprobe oder einem anderen festgelegten Wert entspricht.

Erfindungsgemäß wird also die Analyt-Menge, die einem Normalwert oder sonstigem festgelegtem Wert durch die so genannte "Immunoschwelle" nach dem Titrationsprinzip gebunden und erreicht so nicht mehr die Fängermoleküle im Nachweisfeld. Wenn also beispielsweise 10ng FABP/mL in einer 100microliter Plasma-Probe enthalten sind, werden die absolute Menge von 1 ng FABP, mit den entsprechen Dam-M gebunden, bereits an der Immunoschwelle abgefangen und kann nicht das Nachweisfeld erreichen.

Erfindungsgemäß wird für den Nachweis von niedermolekularen Analyten dem Nachweisfeld ebenfalls eine bevorzugt verdeckte, allerdings anders konstruierte Immunoschwelle vorgelagert. Das Verfahren ist dadurch gekennzeichnet, dass es zum Nachweis niedermolekularer Analyten bevorzugt nach dem Konkurrenzprinzip arbeitet.

Das Analysenelement weist dazu in der Probenaufnahmezone eine definierte Menge an Analyt (A) chemisch fest verbunden mit einem signalgebenden Marker (M) auf (A-M), der adsorptiv gebunden und frei beweglich ist und der sich durch kapillare Kräfte in Richtung Nachweiszone gemeinsam mit dem Analyten in der zu untersuchenden Probe bewegt.

Der Komplex passiert dabei die SAF, die gegen den zu untersuchenden Analyten gerichtet sind und die direkt über Spacer oder über Trägerproteine an das Analysenelement fest gebunden vorliegen. Diese SAF sind so dosiert, dass sie eine festgelegte Menge an A-M binden, so dass
a) bei Normalkonzentration des Analyten in der zu untersuchenden Probe kein Signal in der sichtbaren Nachweiszone erscheint,
b) bei Überschreiten des Normalwertes von Analyt in der Probe dieser mit dem AM um die Bindung am SAF konkurriert und je mehr Analyt sich über normal in der Probe befindet und am SAF binden kann, desto mehr A-M gelangt in die Nachweiszone, wodurch ein Signal entsteht, das der Ag-Konzentration in der Probe äquivalent ist.

Gegenstand der Erfindung sind weiterhin Analysenelemente zum realen Nachweis von hochmolekularen Antigenen in analytischen Proben.

Fig. 1A und 1B stellen beispielsweise Teststreifen für die Benutzung des Sandwichprinzips dar. Sie weisen die folgenden Zonen auf:
- eine sichtbare Probeaufnahmezone (1), die mindestens ein freibewegliches Detektormolekül (Dam) umfasst, der mit einem Marker (M) festverbunden vorliegt (Dam-M), und
- eine sichtbare Nachweiszone (2), welche beide in flüssigkeitsübertragendem Kontakt stehen, wobei diese Zone (2) mindestens ein Affinitätsmolekül zur Bindung des Analyten und eine Indikatorsubstanz zur enthält sowie
- eine vorzugsweise nichtsichtbare Schwellen-Zone (3), die sich zwischen der Probeaufnahmezone (1) und der Nachweiszone (2) befindet und die mindestens einen SAF für den zu bestimmenden hochmolekularen Analyten enthält, der eine definierte Konzentration zur Bindung des Schwellenwertes des zu bestimmenden Analyten aufweist.

Fig. 2A und 2B zeigen ein Analysenelement zum realen Nachweis von niedermolekularen Analyten, vorzugsweise Haptenen in analytischen Proben. Sie stellen beispielsweise einen Teststreifen für die Benutzung des Konkurrenzprinzips dar und umfassen:
- eine sichtbare Probeaufnahmezone (1), die eine definierte Menge des niedermolekularen Analyten mit Markerstoff (M) chemisch fest verbunden (A-M) adsorptiv (also beweglich) aufweist, und
- eine sichtbare Nachweiszone (2), welche beide in flüssigkeitsübertragendem Kontakt stehen, wobei diese Zone (2) mindestens ein Affinitätsmolekül zur Bindung des Analyten enthält, sowie
- eine vorzugsweise nichtsichtbare Schwellen-Zone (3), die sich zwischen der Probeaufnahmezone (1) und der Nachweiszone (2) befindet und die mindestens einen SAF für den niedermolekularen Analyten enthält.

Fig. 3
zeigt ein Analysenelement zum realen Nachweis von niedermolekularen und hochmolekularen Analyten (A), bevorzugt von Antigenen (Ag), in analytischen Proben. Dieser Teststreifen kombiniert das Sandwich - und das Konkurrenzprinzip und umfasst:
- eine sichtbare Probeaufnahmezone (1), die frei bewegliche Detektor-Affinitätsmoleküle (Dam), vorzugsweise Detektorantikörper (DAk) mit Marker (M) fest verbunden (Dam-M) und A-M aufweist, und
- eine sichtbare Nachweiszone (2), welche beide in flüssigkeitsübertragendem Kontakt stehen, wobei diese Zone (2) mehrere verschiedene Fängermoleküle (Fängerantikörper) umfasst, sowie
- eine nichtsichtbare Schwellen-Zone (3), die sich zwischen der Probeaufnahmezone (1) und der Nachweiszone (2) befindet und die mehreren verschiedenen Fängermoleküle umfasst.

Die Vorteile der Erfindung bestehen insbesondere darin:

Der Assay ist billig und sehr einfach zu handhaben.
Er kann mit einer minimalen Probemenge durchgeführt werden, d.h., es werden keine so großen Probemengen wie in ELISAs werden benötigt.
Man hat nach wenigen Minuten bereits das Resultat.
Besonders vorteilhaft ist, daß er von technischen Kräften (z.B Krankenschwestern) oder sogar von Laien durchgeführt werden kann.
Er vermeidet Fehldeutungen (falschpositiv), da er bei Normalwerten absolut kein Signal zeigt.

Die Erfindung soll im folgenden anhand von einem Ausführungsbeispiel näher erläutert werden, ohne sie darauf zu beschränken.

### Beispiel

### Bestimmung von Herz-FABP im Plasma mit einem Teststreifen

Verschiedene Membranen werden überlappend zu einem Teststreifen zusammengefügt. Nitrocellulose wird mit einem Biodot-Gerät: mit folgenden Antikörpermengen in Strichform beschichtet: Schwelle: 0,2µg anti-human Herz-FABP-IgG 2F12 in 10 mM Natriumphosphatpuffer (pH 7,2), Test: 2µg 2F12 in 10 mM Natriumphosphatpuffer (pH 7,2), Kontrolle: 1µg Ziege-anti-Maus IgG in 10 mM Natriumphosphatpuffer (pH 7,2).
Die Antikörper werden an der Nitrocellulose fest gebunden. Das Proben-Auftragungsfeld (0,5 x 1,5 cm) liegt über dem Konjugatfeld (0,5 x 1 cm). Hier werden 5µl mit kolloidalem Gold markierte Detektor-Antikörper aufgetragen adsorptiv gebunden (anti-FABP-IgG F5E10-cgc). Der Teststreifen wird getrocknet. Eine Probe von 80µl Plasma (50 ng FABP/mL) wird aufgetragen.
Die Nitrocellulosemembran hat die Dimension 0,5 x 2,5cm . Am anderen Ende des Streifens befindet sich ein Trägerabsorptionsmaterial von 0,5 x 2cm
Folgende Abstände der Antikörper auf der Nitrocellulose werden eingehalten: 0,8cm für die Schwelle, 1,4cm für den Test, 2cm für die Kontrolle.
Nach 5 min sind sowohl Test- als auch Kontrollstreifen sichtbar. Die vor dem Testfeld befindliche Schwelle wird visuell verdeckt und bindet den Teil des FABP, der dem Normalwert entspricht.

## Patentansprüche

1. Verfahren zum auf Affinitätsreaktion beruhendem verbesserten Nachweis von Analyten wie Antigenen, Antikörpern, Rezeptorliganden, Rezeptoren, DNA und RNA und anderen bioaffinen Molekülen in analytischen biologischen Proben mittels Analysenelementen, insbesondere als Teststreifen, **dadurch gekennzeichnet, dass** zusätzlich zu gebräuchlichen Beschichtungen und konfigurationen mit komplementären Affinitätsmolekülen in der Nachweiszone das Analysenelement zwischen Probenaufnahmezone und Nachweiszone mindestens einen Schwellen-Affinitäts-Fänger (SAF) bestehend aus mindestens einem Fängerantikörper (Schwellen-FAk) gegen mindestens einen Analyten gerichtet für eine vorgelagerte Analyt-Antikörper-Affinitäts-Reaktion aufweist, in einer Konzentration, die den Normalwert des zu bestimmenden Analyten eines Gesunden, einer Umweltprobe oder einen anderen festgelegten Wert eliminiert, wobei die Fängerantikörper auf dem Analysenelement räumlich so angeordnet sind, dass diese Reaktion der Nachweisreaktion als Schwelle vorgelagert abläuft und somit erst über dem Schwellenwert liegende Werte in der Nachweisreaktion sichtbar werden.

2. Verfahren nach Anspruch 1,
**dadurch gekennzeichnet, dass**
als Antigene hochmolekulare Substanzen, wie Proteine, oder niedermolekulare Haptene, wie Pestizide, andere Schadstoffe oder Hormone nachgewiesen werden.

3. Verfahren nach einem der Ansprüche 1 bis 2,
**dadurch gekennzeichnet, dass**
die SAF fest gebunden direkt über Spacersubstanzen oder Trägerproteine auf dem Analysenelement befinden.

4. Verfahren nach einem der Ansprüche 1 bis 3, **dadurch gekennzeichnet,**
**dass** sich SAF als Kreise, Punkt, Ellipsen, und anderen geometrischen Strukturen auf dem Analysenelement befinden.

5. Verfahren nach einem der Ansprüche 1 bis 4,
**dadurch gekennzeichnet, dass** die SAF verdeckt, und somit unsichtbar auf dem Analysenelement angeordnet sind.

6. Verfahren nach einem der Ansprüche 1 bis 4, **dadurch gekennzeichnet**,
als SAF monoklonale oder polyklonale Antikörper oder deren Fragmente oder Fusionsprodukte fungieren, wobei die SAF die gleichen Affinitätsmoleküle sind, die auch im sichtbaren Nachweis-Analysenfeld verwendet werden.

7. Verfahren nach einem der Ansprüche 1 bis 6,
**dadurch gekennzeichnet, dass**
die SAF gegen mindestens einen Analyten gerichtet sind oder gegen mehrere unterschiedliche Analyten.

8. Verfahren nach einem der Ansprüche 1 bis 7,
**dadurch gekennzeichnet, dass**
es zum Nachweis hochmolekularer Analyten (A) nach dem Sandwichprinzip arbeitet, wobei das Analysenelement in der Probenaufnahmezone adsorbierte und somit frei bewegliche Detektoraffinitätsmoleküle (Dam) umfasst, welche mit einem Marker (M) zur Signalbildung chemisch fest verbunden in trockener Form vorliegen, und die nach dem Auftragen der zu untersuchenden flüssigen Probe mit dem zugefügten Analyten einen Analyt-Detektoraffinitätsmolekül-Marker-Komplex (A-Dam-M) bilden, welcher sich durch kapillare Kräfte in Richtung Nachweisfeld bewegt, und dabei die SAF passiert, die gegen den zu untersuchenden Analyten gerichtet sind und die direkt, über Spacersubstanzen oder über Trägerproteine an das Analysenelement fest gebunden vorliegen, wobei die SAF eine definierte Konzentration aufweisen, so dass sie sicher genau die Menge an Analyt (A) in Form von A-Dam-M binden, welche dem Normalwert des Analyten eines Gesunden, einer Umweltprobe oder einem anderen festgelegten Wert entspricht.

9. Verfahren nach einem der Ansprüche 1 bis 7,
**dadurch gekennzeichnet, dass**
es zum Nachweis niedermolekularer Analyten nach dem Konkurrenzprinzip arbeitet, wobei das Analysenelement in der Probenaufnahmezone eine definierte Menge an Analyt (A) chemisch fest verbunden mit einem signalgebenden Marker (M) aufweist (A-M), der adsorptiv gebunden und frei beweglich ist und der sich durch kapillare Kräfte in Richtung Nachweiszone gemeinsam mit dem Analyten in der zu untersuchenden Probe bewegt, dabei die SAF passiert, die gegen den zu untersuchenden Analyten gerichtet sind und die direkt, über Spacer oder über Trägerproteine an das Analysenelement fest gebunden vorliegen und so dosiert sind, dass sie eine festgelegte Menge an A-M binden, so dass
a) bei Normalkonzentration des Analyten in der zu untersuchenden Probe kein Signal in der sichtbaren Nachweiszone erscheint,
b) bei Überschreiten des Normalwertes von Analyt in der Probe dieser mit dem A-M um die Bindung am SAF konkurriert und je mehr Analyt sich über normal in der Probe befindet und am SAF binden kann, desto mehr A-M in die Nachweiszone gelangt, wodurch ein Signal entsteht, das der Ag-Konzentration in der Probe äquivalent ist.

10. Analysenelement zum Nachweis von molekularen Analyten in analytischen Proben, als Teststreifen unter Benutzung des Sandwich- oder/und des Konkurrenzprinzips, umfassend
a) eine sichtbare Probeaufnahmezone (1), die mindestens eine Sorte, frei beweglicher Detektor-Affinitätsmoleküle mit Marker (M) fest verbunden (Dam-M) oder/und eine adsorbierte, definierte Menge des Analyten mit Marker (M) chemisch fest verbunden (A-M) aufweist, und
b) eine sichtbare Nachweiszone (2), welche beide in flüssigkeitsübertragendem Kontakt stehen, wobei diese Zone (2) mindestens eine Sorte von Affinitätsmolekülen zur Bindung des Analyten enthält, sowie
c) eine nichtsichtbare Schwellenzone (3), die sich zwischen der Probeaufnahmezone (1) und der Nachweiszone (2) befindet und die mindestens einen Schwellen-Affinitätsfänger (SAF) für die zu bestimmenden molekularen Analyten enthält.

11. Analysenelement nach Anspruch 10,
**dadurch gekennzeichnet, dass**
es zum realen Nachweis von hochmolekularen Analyten in analytischen biologischen Proben als Teststreifen nach dem Sandwichprinzip aufgebaut ist, wobei
a) es sich bei dem molekularen Analyten um einen hochmolekularen handelt, und
b) die Probeaufnahmezone (1) mindestens eine Sorte von adsorptiv gebundenen Dam-M umfasst, die damit nach Flüssigkeitszugabe frei beweglich sind, und
c) der SAF der Schwellenzone (3) eine definierte Konzentration zur Bindung des Schwellenwertes des zu bestimmenden hochmolekularem Analyten aufweist.

12. Analysenelement nach Anspruch 10,
**dadurch gekennzeichnet, dass**
es zum realen Nachweis von niedermolekularen Analyten in analytischen biologischen Proben als Teststreifen nach dem Konkurrenzprinzip aufgebaut ist, wobei
a) es sich bei dem molekularen Analyten um einen niedermolekularen handelt, und
b) die Probeaufnahmezone (1) eine definierte Menge des niedermolekularen A-M adsorptiv aufweist.

13. Analysenelement nach Anspruch 10,
**dadurch gekennzeichnet, dass**
es zum gleichzeitigen Nachweis von niedermolekularen und hochmolekularen Antigenen in analytischen Proben nach einer Kombination des Sandwich- und des Konkurrenzprinzips arbeitet, wobei
a) es sich bei den molekularen Analyten um niedermolekulare und hochmolekulare Antigene handelt, und
b) Dam-M als Detektorantikörper DAk mit Marker (M) fest verbunden spezifiziert ist, und
c) die Probeaufnahmezone (1) frei bewegliche Dam-M und A-M aufweist, und
d) die Nachweiszone (2) mehrere unterschiedliche Fängerantikörper als Affinitätsmoleküle umfasst, sowie
e) die Schwellenzone (3) mehrere unterschiedliche SAF umfasst.

## Claims

1. A method, based on an affinity reaction, for the improved detection of analytes, such as antigens, antibodies, receptor ligands, receptors, DNA and RNA, and other bio-affinity molecules in analytical biological samples, by means of analyzing elements, preferably test strips, **characterized in that** in addition to customary coatings and configurations with complementary affinity molecules in the detection zone, the analyzing element between sample application zone and analyte detection zone is made up of at least one threshold affinity catcher (TAC) consisting of at least one type of catcher antibody (threshold catcher-ab) directed against at least one type of analyte for a preceding analyte-antibody affinity reaction, in a concentration that eliminates the normal value of the analyte to be detected of a healthy person, of an environmental sample or of another determined value; whereby the catcher-antibodies are spatially arranged on the analyzing element in such a way that this reaction acts as a preceding threshold for the detection reaction , thereby causing only values of the detection reaction above the threshold level to become visible.

2. A method of claim 1, **characterized in that** high-molecular substances, such as proteins, or low-molecular haptens, such as pesticides, other pollutants or hormons are detected as antigens.

3. A method according to claims 1-2, **characterized in that** the TACs are tightly bound to the analyzing element, directly via spacer substances or carrier proteins.

4. A method according to one of claims 1-3, **characterized in that** the TACs are arranged on the analyzing element as circles, dots, ellipses, or other geometrical forms.

5. A method according to one of claims 1-4, **characterized in that** the TACs are covered and thus being arranged unvisible on the analyzing element.

6. A method according to one of claims 1-5, **characterized in that** the TACs are made up of monoclonal or polyclonal antibodies or of fragments or fused products thereof, whereat the TACs are the same affinity molecules, which are also used in the visible analyte detection zone.

7. A method according to one of claims 1-6, **characterized in that** the TACs are directed against at least one analyte or against several different analytes.

8. A method according to one of claims 1-7, **characterized in that** it functions for the detection of high-molecular analytes (A) by way of the sandwich assay technique, whereat the analyzing element comprises, in the sample application zone, adsorbed, and thus free to move, detector affinity molecules (DAM), which are chemically firmly bound to a marker (M), that enables a signal generation, and which are in a dry state, and which form, after application of the liquid sample to be analyzed, an analyte-detector affinity molecule-marker complex (A-DAM-M) with the DAM, which moves, driven by capillary forces, towards the analyte detection zone, thereby passing the TACs, which are directed against the analyte to be detected, and which are directly, by way of a spacer substance or via carrier proteins firmly bound to the analyzing element, whereat the TACs are present in a specified concentration, such that the TACs safely bind the exact amount of analyte (A), by way of forming A-DAM-M, that corresponds to the normal value of a healthy person, an environmental sample or another fixed value.

9. A method according to one of claims 1-7, **characterized in that** it functions for the detection of low-molecular analytes by way of the principle of competition, whereat the analyzing element in the sample application zone comprises a specified amount of analyte (A) chemically firmly bound to a signal-generating marker (M) in a complex (A-M) which is adsorptively bound and free to move and which moves, together with the analyte from the sample to be investigated, by capillary forces towards the analyte detection zone, thereby passing the TACs which are directed against the analyte and which are directly, by way of a spacer substance or via carrier proteins firmly bound to the analyzing element and are dosed in a rate, such that they bind to a specified amount of A-M, so that:
a) at normal concentration of the analyte in the sample to be investigated, there is no signal in the visible detection zone,
b) at exceeding the normal value of analyte in the sample the analyte competes with the A-M for binding to the TACs and the more above-average analyte there is in the sample and binds to the TACs, the more A-M is driven to the analyte detection zone, thereby generating a signal equivalent to the antigen concentration of the sample.

10. Analyzing element for detecting molecular analytes in analytical samples, designed as test strip using the sandwich assay technique or/and the principle of competition, comprising:
a) a visible sample application zone (*1*), containing at least one sort of freely movable detector affinity molecules (DAM) firmly bound to a marker (DAM-M) or/and an adsorbed, specified amount of analyte, firmly chemically bound to a marker (A-M), and
b) a visible analyte detection zone (2), which both are in fluid-conveying contact, whereat this zone (2) contains at least one sort of affinity molecules for binding the analyte, and
c) an invisible threshold zone (3), located between the sample application zone (*1*) and the analyte detection zone (2), and that contains at least one threshold affinity catcher (TAC) directed against the molecular analyte to be determined.

11. Analyzing element according to claim 10, **characterized in that** it is designed as test strip using the sandwich assay technique for detecting high-molecular analytes in analytical biological samples, whereat:
a) the molecular analyte is of high molecular weight, and
b) the sample application zone (*1*) comprises at least one sort of DAM-M, which is adsorptively bound, being therefore free to move after addition of liquid, and
c) the TAC of the threshold zone (3) is in a specified concentration for binding the threshold amount of the high-molecular analyte to be determined.

12. Analyzing element according to claim 10, **characterized in that** it is designed as test-strip using the principle of competition for detecting low-molecular analytes in analytical biological samples, whereat:
a) the molecular analyte is of low molecular weight, and
b) the sample application zone (*1*) provides a specified amount of low-molecular A-M adsorptively bound to it.

13. Analyzing element according to claim 10, **characterized in that** it functions for the simultaneous detection of low- and high-molecular antigens in analytical samples by a combination of the sandwich assay techniqe and the principle of competition, whereat:
a) the molecular analytes are antigens of low and of high molecular weight, and
b) DAM-M is specified as detector antibody (DAb), firmly bound to marker (M), and
c) the sample application zone (*1*) contains freely mobile DAM-M and A-M, and
d) the analyte detection zone (*2*) comprises several different catcher antibodies as affinity molecules, and
e) the threshold zone (*3*) comprises several different TACs.

## Revendications

1. Procédé destiné à améliorer la mise en évidence reposant sur une réaction d'affinité d'analytes tels que les antigènes, les anticorps, les ligands récepteurs, l'ADN et l'ARN et autres molécules bioaffines dans des échantillons biologiques analytiques au moyen d'éléments d'analyse, en particulier de bandes de test, **se caractérisant par le fait qu'**en plus des enductions et configurations usuelles avec des molécules d'affinité complémentaires dans la zone de mise en évidence, l'élément de l'analyse présente entre la zone de prélèvement de l'échantillon et la zone de mise en évidence au moins un capteur d'affinité seuil (SAF) comprenant au moins un anticorps de capture (Fak-seuil), dirigé contre au moins un analyte pour une réaction d'affinité analyte-anticorps en amont, dans une concentration qui élimine la valeur normale de l'analyte à déterminer d'un échantillon sain, d'un échantillon environnement ou une autre valeur déterminée, les anticorps de capture étant disposés dans l'espace sur l'élément d'analyse de sorte que cette réaction se déroule en amont de la réaction de mise en évidence en tant que seuil et ainsi n'étant visibles que par les valeurs supérieures à la valeur-seuil dans la réaction de mise en évidence.

2. Procédé selon la revendication 1,
**se caractérisant par** le fait
des substances à haute densité moléculaire, telles que les protéines, ou des haptènes à faible densité moléculaire telles que les pesticides, d'autres substances toxiques ou des hormones sont mises en évidence en tant qu'antigènes.

3. Procédé selon l'une des revendications 1 à 2,
**se caractérisant par** le fait
les SAF se trouvent attachés directement sur l'élément d'analyse via des substances-espaceurs ou des protéines porteuses.

4. Procédé selon l'une des revendications 1 à 3, **se caractérisant par le fait que** les SAF se trouvent sur l'élément d'analyse sous forme de cercles, de points, d'ellipses et d'autres structures géométriques.

5. Procédé selon l'une des revendications 1 à 4,
**se caractérisant par** le fait
les SAF sont disposés sur l'élément d'analyse de façon cachée et ainsi invisibles.

6. Procédé selon l'une des revendications 1 à 4, **se caractérisant par le fait que** des anticorps monoclonaux ou polyclonaux ou leurs fragments ou leurs produits de fusion jouent le rôle de SAF, les SAF étant les mêmes molécules d'affinité qui sont aussi utilisées dans le champ d'analyse à mise en évidence visible.

7. Procédé selon l'une des revendications 1 à 6,
**se caractérisant par** le fait
les SAF sont orientés contre au moins un analyte ou contre plusieurs analytes différents.

8. Procédé selon l'une des revendications 1 à 7,
**se caractérisant par le fait**
**qu'**il travaille pour mettre en évidence des analytes à haute densité moléculaire (A) selon le principe sandwich, l'élément d'analyse englobant les molécules d'affinité détectrices (Dam) absorbées dans la zone de capture de l'échantillon, ainsi à mobilité libre, molécules qui se trouvent sous forme sèche, liées chimiquement avec un marqueur (M) pour former le signal, et qui forment, après avoir appliqué l'analyte ajouté à l'échantillon liquide à examiner, un complexe analyte-molécule d'affinité détectrice-marqueur (A-Dam-M) qui se déplace en direction du champ de mise en évidence par le biais des forces capillaires, et qui traverse ainsi les SAF qui sont dirigés contre les analytes à examiner et qui se trouvent attachés directement à l'élément d'analyse via des substances-expaceurs ou des protéines porteuses, les SAF présentant une concentration définie de sorte qu'ils lient certainement exactement la quantité d'analyte (A) sous forme de A-Dam-M qui correspond à la valeur normale de l'analyte d'un échantillon sain, d'un échantillon d'environnement ou à une autre valeur déterminée.

9. Procédé selon l'une des revendications 1 à 7,
**se caractérisant par le fait**
**qu'**il travaille pour mettre en évidence des analytes à faible densité moléculaire selon le principe de concurrence, l'élément d'analyse présentant dans la zone de capture de l'échantillon une quantité d'analyte (A) liée chimiquement (A-M), avec un marqueur signalisateur (M) qui est lié en corps absorbé et a une mobilité libre et qui se déplace conjointement avec l'analyte dans l'échantillon à examiner par le biais de forces capillaires en direction de la zone de mise en évidence tout en traversant les SAF qui sont orientés contre les analytes à examiner et qui se trouve liés directement à l'élément d'analyse via des espaceurs ou des protéines porteuses et qui sont dosés de sorte qu'elles lient une quantité déterminée de A-M de sorte que
a) en cas de concentration normale de l'analyte dans l'échantillon à examiner, aucun signal ne paraît dans la zone de mise en évidence visible,
b) en cas de dépassement de la valeur normale d'analyte dans l'échantillon, ce dernier concurrence avec l'A-M pour la liaison au SAF et plus il y a d'analyte au-dessus de la normal dans l'échantillon et est en mesure de lier sur le SAF, plus d'A-M accède à la zone de mise en évidence, ce qui provoque un signal qui est équivalent à la concentration Ag dans l'échantillon.

10. Élément d'analyse pour mettre en évidence des analytes moléculaires dans des échantillons analytiques en tant que bandes de test en utilisant le principe sandwich et/ou de concurrence englobant
a) une zone de capture de l'échantillon visible (1) qui présente au moins une sorte, des molécules d'affinité détectrice à mobilité libre avec marqueur (M) lié (Dam-M) ou/et une quantité définie, absorbée de l'analyte lié chimiquement (A-M) avec le marqueur (M) et
b) une zone de mise en évidence visible (2) qui toutes deux sont en contact de transfert liquide, cette zone (2) contenant au moins une sorte de molécule d'affinité pour la liaison de l'analyte, ainsi que
c) une zone seuil non visible (3) qui se trouve entre la zone de capture de l'échantillon (1) et la zone de mise en évidence (2) et qui contient au moins un capteur d'affinité de seuil (SAF) pour les analytes moléculaires à définir.

11. Élément d'analyse selon la revendication 10,
**se caractérisant par le fait**
**qu'**il est structuré pour la mise en évidence réelle d'analytes à haute densité moléculaire dans des échantillons biologiques analytiques en tant que bandes de test selon le principe sandwich,
a) l'analyte moléculaire étant un analyte à haute densité moléculaire et
b) la zone de capture de l'échantillon (1) englobant au moins une sorte de Dam-M lié comme corps absorbée qui est ainsi de mobilité libre après l'ajout de liquide et
c) le SAF de la zone seuil (3) présentant une concentration définie pour la liaison de la valeur seuil de l'analyte à haute densité moléculaire à définir.

12. Élément d'analyse selon la revendication 10,
**se caractérisant par le fait**
**qu'**il est structuré pour la mise en évidence réelle d'analytes à haute densité moléculaire dans des échantillons biologiques analytiques en tant que bandes de test selon le principe sandwich,
a) l'analyte moléculaire étant un analyte à faible densité moléculaire et
b) la zone de capture d'échantillon (1) présentant sous forme de corps absorbé une quantité définie de A-M à faible densité moléculaire.

13. Élément d'analyse selon la revendication 10,
**se caractérisant par le fait**
**qu'**il travaille selon une combinaison du principe sandwich et du principe de concurrence pour la mise en évidence simultanée d'antigènes à faible densité moléculaire et à haute densité moléculaire,
a) l'analyte moléculaire étant un analyte à faible densité moléculaire et à haute densité moléculaire et
b) Dam-M étant spécifié en tant qu'anticorps détecteur DAk lié avec un marqueur (M) et
c) la zone de capture d'échantillon (1) présentant Dam-M et A-M à mobilité libre et
d) la zone de mise en évidence (2) englobant plusieurs anticorps capteurs différents en tant que molécule d'affinité ainsi que
e) la zone seuil (3) englobant plusieurs SAF différents.
